# EUROPEAN PATENT SPECIFICATION

(11) **EP 2 713 776 B1**
(45) Date of publication and mention of the grant of the patent: **22.07.2015**
(21) Application number: 12735013.0
(22) Date of filing: 01.06.2012
(51) Int. Cl.: A61K 31/05, A61K 31/198, A61K 31/205, A61K 31/355, A61K 31/4045, A61K 31/519, A61K 33/04, A61K 45/06, A61P 15/08, A23L 1/29, A23L 1/30

(54) **ANTIOXIDANT COMPOSITION FOR THE REDUCTION OF THE CAUSES OF REDUCED MALE REPRODUCTIVE CAPACITY**
ANTIOXIDATIONSZUSAMMENSETZUNG ZUR REDUZIERUNG DER URSACHEN VON VERRINGERTER MÄNNLICHER FORTPFLANZUNGSFÄHIGKEIT
COMPOSITION D'ANTIOXYDANT POUR LA RÉDUCTION DES CAUSES DE CAPACITÉ RÉDUITE DE REPRODUCTION MÂLE

(30) Priority: 03.06.2011 IT MI20111010
(43) Date of publication of application: 09.04.2014
(73) Proprietor: LO. LI. Pharma S.r.l., 00156 Roma (IT)
(72) Inventor: UNFER, Vittorio, I-00155 Roma (IT)
(74) Representative: Brighenti, Livio
(86) International application number: PCT/IB2012/052770
(87) International publication number: WO 2012/164535

(56) References cited:
- WO-A1-2005/067972
- DE-U1-202008 016 372
- US-A- 5 922 704
- US-A1- 2006 128 714

## Description

### Field of the invention

The object of the present invention is an antioxidant composition useful for the reduction of the causes of reduced male reproductive capacity.

### State of the art

In recent decades we have seen a marked increase in the rate of combined infertility and, in particular, an increase in male factor infertility. Around 30-35% of infertile couples are affected by oligoasthenoteratozoospermia, i.e. a reduction in the number and in the motility of the spermatozoa and alteration of the morphology thereof. The causes of this condition are still unknown; may therapies have been proposed to remedy this problem, but they have often proved ineffective.

In recent years, research into the causes of male infertility has addressed the study of oxidative stress, which appears to play a fundamental role in the onset of this condition. Large part of cellular oxidative stress is mediated by free radicals, in particular by reactive oxygen species (ROS) and reactive nitrogen species (RNS), molecules equipped with high chemical reactivity by virtue of the presence of unpaired valence electrons.

Most of the cells are provided with endogenous antioxidant systems both of the enzymatic type such as superoxide dismutase, catalase, glutathione peroxidase and glutathione reductase, and of the non-enzymatic type such as vitamin C, vitamin E, coenzyme Q10, etc., which are in general able to effectively combat the oxidative action of ROS and RNS. However, when these defences are not sufficient, an excess of reactive species, which can promote pathological damage, accumulates in the cells oxidising the lipids of the cellular membranes, the proteins and the DNA, thus causing even very significant damage to cell functionality.

In the specific case of male germ cells, a certain amount of free radicals produced by the spermatozoa is important for the maturation process thereof and for making them capable of fertilization. On the other hand, it must be borne in mind that the spermatozoa, losing the cytoplasm during maturation, are lacking most of the defensive mechanisms that characterise the other cellular species and are therefore more susceptible to the alterations caused by the negative effects of the oxidative reactions. The result is therefore that ROS and RNS are accumulated in high numbers by spermatozoa with morphological alterations.

The radicals in excess, many of which are produced by the leucocytes present in the seminal plasma and by the immature spermatozoa prematurely released by the seminiferous tubules, subject the male gametes to an oxidative stress, in some cases altering the morphological characteristics thereof (head or tail structure), but above all negatively influencing the stability of the genetic code. In addition, the spermatozoa are particularly sensitive to the action of the radicals due to the composition of their plasmatic membrane that is rich in polyunsaturated fatty acids, which are easily oxidised by the free radicals.

Thus from a practical point of view, the oxidative stress that attacks the male gametes is capable of altering both the motility and the morphology of the cells and the stability of the DNA, and of altering the normal structure of the mitochondria.

All these conditions cause a reduced reproductive capacity, early spontaneous abortions and in certain cases, genetic mutations of the embryo. The factors capable of inducing an oxidative stress in the seminal fluid are multiple: reproductive system conditions (variocele, prostatitis), particular lifestyles (smoke, alcohol, drugs), environmental and food pollution (radiations, smog, industrial gases, unbalanced diet rich in fats) represent the main sources of stress for spermatozoa.

In the light of the high percentage of people affected by male infertility, there is therefore a need to find substances capable of improving the condition. Since the role of the reactive oxygen species in increasing infertility has been proven, the use of substances having an antioxidant action is object of a growing interest.

US 5 922 704 discloses a nutritional supplement for men comprising inositol, NAC, vitamin E. folic acid, selenium.

DE 20 2008 016372 describes a vitamin-containing composition for the nutrittive balanced supplementation of the nutrition for inner ear diseases comprising inositol, NAC, vitamin E, folic acid, selenium. L-carnitin.

WO 2005/067972 reports a niutrient composition for increasing immune strenght comprising inositol, NAC, vitamin E and selenium.

US 2006/128714 describes multi-nutrient supplements comprising NAC, myo-inositol, folica acid and Vitamin E.

### Summary of the invention

The main objective of this invention is to provide a composition capable of improving the reproductive capacity of men.

Within the scope of this objective, one aim of the invention is to produce a composition capable of reducing the causes of reduced male reproductive capacity. In particular, one aim of the invention is to form a composition capable of promoting an increase in the amount of the spermatozoa and an improved quality thereof.

This objective, and these and other aims that will become clearer below, are achieved by a composition comprising inositol and N-acetyl cysteine. The aforementioned objective and aims are also achieved by the composition described herein for use in the treatment of the conditions that cause a reduction in male fertility.

Further aims, characteristics and advantages of the invention will become clearer from the description of one preferred, but non-exclusive, embodiment of the composition according to the invention.

### Detailed description of the invention

One aspect of the invention relates to a composition comprising inositol and N-acetyl cysteine.

Inositol is chemically a cyclic polyol having the function of vitamin factor. It is part of the vitamin B complex and is involved in calcium-dependent intracellular signalling pathways and in cellular signal transduction through the plasmatic membrane. It regulates various cellular process including cellular differentiation and proliferation through the release of calcium and it also part of the composition of membrane phospholipids. Inositol is capable of producing stimulating effects both on a motor level and on the central nervous system; it is used in muscular dystrophy therapy and in hepatopathies. Furthermore, it controls the secretion of certain endocrine glands including the gonads. Its natural sources are untreated unprocessed wholegrain cereals, citrus fruits, brewer's yeast. As regards its effect on male fertility, its involvement in the maturation and in the migration of spermatozoa from the epididymis has recently been hypothesised. In addition, inositol has proved important in stabilising tubulin, a protein that is fundamental for spermatozoon movement

N-acetyl cysteine (NAC) is known for its antioxidant properties; in addition, the inventors have carried out a study with the aim of verifying the existence of an advantageous effect of NAC on seminal parameters in cases of idiopathic male infertility. As previously mentioned, the production of reactive species, of reactive oxygen and nitrogen species in particular, is a normal physiological even in various organs. Nevertheless, the overproduction of reactive species is harmful to the spermatozoa and has been associated with increased male sterility. In this study, 120 patients were randomly divided into two groups. The study group (60 men) was treated for 3 months with NAC (600 mg/day by mouth), the control group (60 men) was treated for 3 months with placebo. None of the parameters analysed in the study (spermatozoa motility, volume and viscosity of the seminal fluid, plasma redox status) differed significantly among the members of the two groups at the time of trial recruitment. The redox status was determined by measuring total antioxidant capacity, total peroxide and the oxidative stress index in the plasma samples. The semen parameters were assessed following treatment with NAC and were compared with the baseline values and those of the control group. The results achieved have shown that the administration of NAC had effects on the improvement of spermatozoa motility, the volume and viscosity of the seminal fluid. Following treatment with NAC, the total antioxidant capacity of the serum was greater and, at the same time, a significant reduction of the total oxidative stress was observed in the group treated with NAC compared to the control group. These positive effects have the result of reducing the reactive oxygen and/or nitrogen species in the serum and of decreasing the viscosity of the seminal fluid.

The inventors of the present invention have surprisingly discovered that the composition according to the invention comprising inositol and N-acetyl cysteine guarantees a synergistic effect. With the expression "synergistic effect" we mean that the effect mediated by the composition according to the invention is greater than the sum of the effects, e.g. antioxidant effects, guaranteed by the individual components when considered separately.

As well as inositol and N-acetyl cysteine, the composition of the present invention can comprise one or more from among melatonin, Vitamin E, L-carnitine, L-arginine, folic acid and selenium.

Melatonin is known for it capacity to decrease oxidative stress through its free radical scavenger action. In particular, melatonin is effective in combating the effect of the reactive oxygen species (ROS) and of the reactive nitrogen species (RNS).

Melatonin is also involved in the modulation of testicular function. A functional interaction between testosterone and melatonin has been identified in the epididymis, the part of the male reproductive system that is important for the maturation and the storage of the spermatozoa before they pass into the female reproductive tract. Various studies have concluded that melatonin improves semen quality and that its administration is useful for increasing reproductive capacity.

Vitamin E is capable of increasing the vitality of the spermatozoa thanks to is now known antioxidant properties which, by allowing oxidative damage to me significantly limited, allow the spermatozoa to acquire greater resistance, retain their physiological functions for longer and have greater possibilities of fertilization.

L-carnitine is an amino acid synthesised by the human body in the liver and in the kidneys from lysine and methionine in the presence of vitamins B6, C and iron. L-carnitine plays an important role for the motility of the spermatozoa; it is in fact present in high concentrations in male seminal fluid, in which it plays an important role in the energy metabolism of the spermatozoa. The concentration of L-carnitine in the seminal fluid is linked to semen quality. Clinical studies carried out by the inventors of the present composition show that supplementing with L-carnitine over a period of 3 to 6 months can positively modify semen concentration and count. and the percentage of spermatozoa having straight-line progression. Supplementation with L-carnitine is recommended for individuals that wish to take care of their reproductive health.

L-arginine is an amino acid that is present in the proteins of all forms of life. The main function of L-arginine in the human body is to produce nitric acid, a substance of great importance in the normal sexual functions of both men and women. Nitric oxide is a neurotransmitter implicated in various physiological processes, one of which is the increase in the circulation of the blood in the penis during arousal. An insufficient influx of blood is the main cause of total impotence in men. L-arginine improves the influx of blood into the genital area and facilitates more intense, longer-lasting and more frequent erections. A similar effect is also produced in women: a greater influx of blood makes the clitoral and vaginal tissue more sensitive and responsive to stimulation, facilitating orgasms. L-arginine benefits the production and the health of spermatozoa, in fact 80% of seminal fluid consists of arginine. Men with a low concentration of spermatozoa in their semen (oligospermia) have seen an increase in fertility and in the volume of ejaculations after taking arginine.

It has also been known for some time that during pregnancy, there is a correlation between the amount of folic acid taken by the mother and the reduction of the risk of the occurrence of malformations in the child; nevertheless, the father could also be involved in the health of the newborn.

One study published on *Human Reproduction* (Young S.S. et al., The association of folate, zinc and antioxidant intake with sperm aneuploidy in healthy non-smoking men. Hum Reprod. 2008 May;23(5):1014-22. Epub 2008 mar 19) indicates that paternal diet could reduce the occurrence of aneuploidy (alteration of the number of chromosomes) in the spermatozoa. It has in fact been seen that 1 to 4% of the spermatozoa of healthy subjects present this type of anomaly, with the consequent risk of the birth of children affected by Down's Syndrome, Turner's Syndrome or Klinefelter's Syndrome

In the above study, the semen of 89 healthy non-smoking subjects was analysed, for whom the intake of certain nutrients, including, in addition to folic acid, zinc, vitamin C, vitamin E and beta-carotene, was assessed while taking into account the amount provided by both the diet and dietary supplements. This study has shown the existence of a statistical correlation between the intake of high amounts of folates and a low number of spermatozoa with aneuploidy. In fact in the men who were taking folic acid there was a 20 to 30% reduction in spermatozoa with alterations in the number of chromosomes. In conclusion, in healthy and non-smoking subjects the administration of folic acid could be linked to a decrease in the incidence of spermatozoa affected by aneuploidy.

Selenium is an essential mineral that is found in minimal qualities in the human body. The mineral exists in the form of inorganic sodium selenite, sodium selenate and organic selenium. It is a natural antioxidant that carries out its protective action against free radicals and maintains the elasticity of the tissues that tend to lose elasticity as a result of ageing. The cells of male semen contain large amounts of selenium that are lost in the course of sexual relations, which is the reason that selenium requirements are higher for men than for women. Selenium is essential for reproduction. Studies carried out by the inventors have shown that rats fed a low-selenium diet recorded a progressive reduction in the quality of the semen (immobile spermatozoa and with fractures at the level of the mitochondrial collar) to arrive at a complete testicular atrophy. This condition proved to be completely reversible by re-including selenium in the diet.

The inventors of the present invention have surprisingly discovered that the composition according to the invention comprising inositol and N-acetyl cysteine guarantees a synergistic antioxidant effect. With the expression "synergistic effect" we mean that the antioxidant effect mediated by the composition according to the invention is greater than the sum of the antioxidant effects guaranteed by the individual components when considered separately.

The components of the composition according to the invention are present in the composition itself according to the invention on the basis of a preset content, in particular:
- the inositol is present in an amount comprised between 500 and 4000 mg; and
- the N-acetyl cysteine is present in an amount comprised between 200 and 1000 mg.

More preferably, within the composition according to the present invention, the amount of inositol can be of between 500 and 2000 mg and the amount of N-acetyl cysteine can be of between 400 and 800 mg. Even more preferably, the amount of inositol can be of between 500 and 1500 mg.

In another embodiment, the composition according to the invention can further comprise, in addition to inositol and N-acetyl cysteine, one or more antioxidant properties. Preferably, said one or more antioxidant components can be selected from the group consisting of melatonin, Vitamin E, L-carnitine, L-arginine, folic acid and selenium.

In one preferred embodiment, Vitamin E, L-carnitine, L-arginine, folic acid and selenium can be present in the composition in amounts falling within the following ranges:
- melatonin: between 1 and 5 mg;
- Vitamin E: between 5 and 36 mg;
- L-carnitine: between 15 and 200 mg;
- L-arginine: between 15 and 200 mg;
- folic acid: between 100 and 400 µg;
- selenium: between 20 and 55 µg.

More preferably, in the composition according to the invention the aforementioned components can be contained in the following amounts:
- melatonin: between 2 and 5 mg;
- Vitamin E: between 20 and 36 mg;
- L-carnitine: between 20 and 100 mg;
- L-arginine: between 20 and 100 mg;
- folic acid: between 100 and 250 µg;
- selenium: between 30 and 55 µg.

In the composition according to the invention, inositol can be present in one or more of the isomeric forms selected from the group comprising myo-inositol, scyllo-inositol, muco-inositol, D-chiro-inositol, neo-inositol, L-chiro-inositol, allo-inositol, epi-inositol and cis-inositol. Preferably, inositol is present in the composition in its isomeric form identified as myo-inositol.

In another aspect, the invention relates to the composition according to the invention for use in the treatment of conditions that cause reduced male reproductive capacity. For example, oligospermia, asthenospermia, teratospermia, azoospermia and oligoasthenoteratozoospermia can be cited among the conditions associated with reduced male reproductive capacity.

In particular, the composition of the invention can be used to treat the conditions of low male reproductive capacity, improving the quality of the seminal fluid and consequently increasing male fertility. In one preferred embodiment, the composition according to the invention can be used to improve the quality of the seminal fluid by decreasing the amount of substances having an oxidant action present within the testis and the seminal fluid itself. In particular, the oxidising substances, the amount of which can be reduced by the composition in question, are the free radicals of oxygen and of nitrogen (respectively ROS, reactive oxygen species, and RNS, reactive nitrogen species).

In one preferred embodiment, the composition according to the invention can reduce the level of reactive oxygen species. In another preferred embodiment, the composition can reduce the level of reactive nitrogen species. It follows that the composition according to the invention, for use in improving the quality of seminal fluid, allows the spermatozoa to be protected from the oxidative stress caused by ROS and RNS. By virtue thereof, the spermatozoa more easily maintain the morphological and functional characteristics most in line with the exercising of the reproductive function. ROS and RNS are in fact physiologically generated in all the cells, including the spermatozoa, as products of the normal cellular metabolism. Nevertheless, while in the other cells their level is maintained constant by enzymatic or non-enzymatic mechanisms capable of eliminating them and/or of combating the oxidation processes mediated by them, in the spermatozoa, ROS and RNS can accumulate in excess, in that while losing the cytoplasm during cellular maturation, said cells lack some of these protection mechanisms against oxidising chemical species. The free radicals of oxygen and nitrogen are therefore among the main culprits for damaging the cellular structure of spermatozoa. In particular, the oxidative damage at the level of the polyunsaturated fatty acids of the plasmatic membrane can be cited, which can lead to morphological alterations (teratospermia) or alterations of cell motility (asthenospermia).

The composition of the invention can therefore be used to combat the presence of reactive oxygen and nitrogen species within the testis and the seminal fluid, by increasing the amount of spermatozoa and the quality thereof in terms of the morphology, motility and integrity of the gene pool. In addition, as previously mentioned, the components of the composition according to the invention operate synergistically in treating the conditions that cause reduced male fertility, in particular promoting the decrease of the oxidising substances present in the testis and in the seminal fluid.

To verify the efficacy of the present composition, the inventors carried out a study over 45 days on a homogenous group of 32 patients. At the start of the trial, the semen of each of the 32 patients was subjected to analysis by optical microscope through which the motility and concentration of the spermatozoa in the seminal fluid and the percentage content of abnormal spermatozoa were assessed.

The total of the sample was then subdivided into three groups: one group comprising 12 patients (group A) and two groups each comprising 10 patients (groups B and C).

Patients in group A were administered the composition according to the invention, comprising 1000 mg of inositol and 600 mg of N-acetyl cysteine.

Patients in group B were administered a composition comprising 1000 mg of inositol, but not comprising N-acetyl cysteine.

Patients in group C were administered a composition comprising 600 mg of N-acetyl cysteine, but not comprising inositol.

At the end of the 45 days of administration, the analyses were repeated on the sperm of all the patients, both of the study group (A) and the control groups (B) and (C). The results achieved are listed shown in the following table:

| | group A (inositol + NAC) | group B (inositol) | group C (NAC) |
|---|---|---|---|
| spermatozoa motility | + 40% | + 25% | + 8% |
| spermatozoa concentration | + 180% | + 110% | + 30% |
| percentage of abnormal spermatozoa | - 55% | - 28% | - 16% |
| presence of ROS and RNS | -34 % | -8,00% | -17,00% |

The data contained in the table shows the synergistic action of the composition according to the invention (administered to group (A)) in improving the various parameters associated with the quality of the seminal fluid. In particular, it is observed that the motility and the concentration of the spermatozoa within the seminal fluid have undergone a percentage increase, in patients of group (A), greater than the sum of the increases measured for the subjects in groups (B) and (C).

Similarly, it is observed that the "negative" parameters, such as the percentage of abnormal spermatozoa and the level of reactive oxygen and nitrogen species have decreased, in patients in group (A), by a percentage greater than the sum of the decreases recorded for the subjects in groups (B) and (C).

All the variations observed have been statistically significant, recording a significance level *(p-value)* of less than 0.01.

It has therefore been shown that the composition according to the present invention is effective in improving reproductive functionality in subjects affected by conditions that reduce their fertility, such as oligospermia, asthenospermia, teratospermia, azoospermia or oligoasthenoteratozoospermia.

Another aspect of the present invention relates to the use of the herein described composition as a nutritional supplement. The composition according to the invention in fact promotes the intake of biologically active substances that are often not produced in sufficient amounts by the body or are not taken in sufficient amounts with the diet. As such, the composition pursuant to this invention can be administered to increase the intake of substances having an antioxidant action in the body.

In the light of the foregoing, it has in practice been stated how the composition according to the invention fully achieves the preset objective in that, thanks to the high antioxidant power, it is able to protect the spermatozoa from the harmful action of the reactive oxygen and nitrogen species, thus increasing male reproductive capacity. Preferably, the composition achieves the preset objective through the synergistic antioxidant effect of its components.

Moreover, the composition pursuant to the invention can be advantageously used to increase the reproductive functionalities by subjects who wish to increase their possibilities of conceiving.

Although the composition according to the invention has been designed in particular for the improvement of male reproductive capacity, it can nevertheless find application, more generally, in the treatment and in the prevention of the conditions associated to an excessive presence of free radicals, reactive oxygen and nitrogen species and/or oxidising substances in general.

## Claims

1. Antioxidant composition comprising inositol and N-acetyl cysteine wherein the inositol is present in an amount comprised between 500 and 4000 mg and the N-acetyl cysteine is present in an amount comprised between 200 and 1000 mg.

2. Composition according to claim 1 that further comprises one or more antioxidant components selected from the group consisting of melatonin, vitamin E, L-carnitine, L-arginine, folic acid and selenium.

3. Composition according to claim 2, wherein melatonin is present in an amount comprised between 1 and 5 mg, Vitamin E is present in an amount comprised between 5 and 36 mg, L-carnitine is present in an amount comprised between 15 and 200 mg, L-arginine is present in an amount comprised between 15 and 200 mg, folic acid is present in an amount comprised between 100 and 400 µg and selenium is present in an amount comprised between 20 and 55 µg.

4. Composition according to any one of claims 1 to 3 wherein inositol is selected from the group that consists of myo-inositol, scyllo-inositol, muco-inositol, D-chiro-inositol, neo-inositol, L-chiro-inositol, allo-inositol, epi-inositol and cis-inositol.

5. Composition according to claim 4 wherein inositol is myo-inositol.

6. Composition according to any one of claims 1-5, for use as a medicine.

7. Composition according to any one of claims 1-6, for use in the treatment of conditions that cause reduced male reproductive capacity.

8. Composition for use in the treatment according to claim 7, by improving the quality of the seminal fluid.

9. Composition for use in the treatment according to claim 8, wherein the improvement of the quality of the seminal fluid is due to the decrease in the oxidising substances present within the testis and the seminal fluid.

10. Composition for use in the treatment according to claim 9, wherein the oxidising substances are reactive oxygen and nitrogen species.

11. Composition for use in the treatment according to claim 7, wherein the condition that causes a reduced male reproductive capacity is a condition selected from the group consisting of oligospermia, asthenospermia, teratospermia, azoospermia and oligoasthenoteratozoospermia.

12. Use of the composition according to claims 1-6 as a dietary supplement.

## Patentansprüche

1. Antioxidationszusammensetzung umfassend Inositol und N-Acetylcystein, wobei das Inositol in einer Menge zwischen 500 und 4000 mg vorhanden ist und das N-Acetylcystein in einer Menge zwischen 200 und 1000 mg.

2. Zusammensetzung gemäß Anspruch 1, die weiter einen oder mehrere Antioxydations-komponenten umfasst, die aus der Gruppe bestehend aus Melatonin, Vitamin E, L-Carnitin, L-Arginin, Folsäure und Selen ausgewählt sind.

3. Zusammensetzung gemäß Anspruch 2, bei welcher Melatonin in einer Menge zwischen 1 und 5 mg, Vitamin E in einer Menge zwischen 5 und 36 mg, L-Carnitin in einer Menge zwischen 15 und 200 mg, L-Arginin in einer Menge zwischen 15 und 200 mg, Folsäure in einer Menge zwischen 100 und 400 µg und Selen in einer Menge zwischen 20 und 55 µg vorhanden ist.

4. Zusammensetzung nach einem der Ansprüche 1 bis 3, bei welcher das Inositol aus einer Gruppe ausgewählt ist, die Myo-Inositol, Scyllo- Inositol, Muco- Inositol, D-Chiro-Inositol, Neo- Inositol, L-Chiro-Inositol, Allo- Inositol, Epi- Inositol, und Cis- Inositol aufweist.

5. Zusammensetzung gemäß Anspruch 4, wobei Inositol Myo-Inositol ist.

6. Zusammensetzung nach einem der Ansprüche 1 bis 5, für die Verwendung als Medikament.

7. Zusammensetzung nach einem der Ansprüche 1 bis 6, für die Verwendung in der Behandlung von Zuständen, die eine verminderte männliche Fortpflanzungsfähigkeit zur Folge haben.

8. Zusammensetzung für die Verwendung zur Behandlung gemäß Anspruch 7, wobei die Qualität der Samenflüssigkeit verbessert wird.

9. Zusammensetzung für die Verwendung zur Behandlung gemäß Anspruch 8, wobei die Verbesserung der Qualität der Samenflüssigkeit auf der Verminderung von oxidierenden Substanzen in den Hoden und in der Samenflüssigkeit beruht.

10. Zusammensetzung für die Verwendung zur Behandlung gemäß Anspruch 9, wobei die oxidierenden Substanzen reaktive Sauerstoff- und Stickstoffverbindungen sind.

11. Zusammensetzung für die Verwendung zur Behandlung gemäß Anspruch 7, wobei die Zustände, die eine verminderte männliche Fortpflanzungsfähigkeit zur Folge haben , ein Zustand ist, der aus der Gruppe Oligospermie, Asthenospermie, Teratospermie, Azoospermie und Oligoastheoteratozoospermie ausgewählt ist.

12. Verwendung der Zusammensetzung gemäß den Ansprüchen 1 bis 6 als Nahrungsergänzungsmittel.

## Revendications

1. Composition d'antioxydant comprenant de l'inositol et de la N-acétylcystéine, dans laquelle l'inositol est présent dans une quantité comprise entre 500 et 4 000 mg et la N-acétylcystéine est présente dans une quantité comprise entre 200 et 1000 mg.

2. Composition selon la revendication 1, qui comprend en outre un ou plusieurs composants antioxydants choisis dans le groupe consistant en la mélatonine, la vitamine E, la L-carnitine, la L-arginine, l'acide folique et le sélénium.

3. Composition selon la revendication 2, dans laquelle la mélatonine est présente dans une quantité comprise entre 1 et 5 mg, la vitamine E est présente dans une quantité comprise entre 5 et 36 mg, la L-carnitine est présente dans une quantité comprise entre 15 et 200 mg, la L-arginine est présente dans une quantité comprise entre 15 et 200 mg, l'acide folique est présent dans une quantité comprise entre 100 et 400 µg et le sélénium est présent dans une quantité comprise entre 20 et 55 µg.

4. Composition selon l'une quelconque des revendications 1 à 3, dans laquelle l'inositol est choisi dans le groupe consistant en le myo-inositol, le scyllo-inositol, le muco-inositol, le D-chiro-inositol, le néo-inositol, le L-chiro-inositol, l'allo-inositol, l'épiinositol et le cis-inositol.

5. Composition selon la revendication 4, dans laquelle l'inositol est le myo-inositol.

6. Composition selon l'une quelconque des revendications 1 à 5, pour son utilisation comme remède.

7. Composition selon l'une quelconque des revendications 1 à 6, pour son utilisation dans le traitement d'affections qui provoquent une capacité de reproduction masculine réduite.

8. Composition pour son utilisation dans le traitement selon la revendication 7, par amélioration de la qualité du liquide séminal.

9. Composition pour son utilisation dans le traitement selon la revendication 8, dans laquelle l'amélioration de la qualité du fluide séminal est due à la diminution des substances oxydantes présentes au sein des testicules et du fluide séminal.

10. Composition pour son utilisation dans le traitement selon la revendication 9, dans laquelle les substances oxydantes sont des espèces oxygénées azotées réactives.

11. Composition pour son utilisation dans le traitement selon la revendication 7, dans laquelle l'affection qui provoque une capacité de reproduction masculine réduite est une affection choisie dans le groupe consistant en l'oligospermie, l'asthénospermie, la tératospermie, l'azoospermie et l'oligoasthénotératozoospermie.

12. Utilisation de la composition selon les revendications 1 à 6, comme complément alimentaire.
